# EUROPEAN PATENT APPLICATION

(11) **EP 1 618 885 A1**
(43) Date of publication of application: **25.01.2006**
(21) Application number: 04016288.5
(22) Date of filing: 10.07.2004
(51) Int. Cl.: A61K 36/49, A61K 31/00, A61K 31/201, A61P 9/14, A61P 17/00

(54) **Cosmetic and/or pharmaceutical compositions containing fatty acids and extracts of castanea sativa**

(71) Applicant: Cognis IP Management GmbH, 40589 Düsseldorf (DE)
(72) Inventor: Rull Prous, Santiago, 08034 Barcelona (ES)

(57) **Abstract**

Suggested are cosmetic and/or pharmaceutical compositions comprising
(a) physiologically active fatty acids, their salts and their esters, and
(b) extracts of *Castanea sativa* or its active principles,
said mixtures being active particularly for fighting cellulites.

## Description

### Object of the invention

The present invention relates to the area of cosmetic or pharmaceutical compositions and more particularly refers to new compositions for topical or oral administration comprising combinations of physiological active fatty acids or their derivatives and certain plant extracts or their active principles.

### State of the art

The term "cellulite" was coined in 1973 to refer to adipose or fat tissue trapped in honeycomblike connective tissue. This appearance is much more common in women than in men because of differences in the way fat, muscle, and connective tissue are distributed in men and women's skin. Cellulite results in uneven bulging of the skin, leading to the unsightly "cottage cheese" or dimpled "orange peel" look. Although female hormones may play a role in contributing to this fat distribution, cellulite is not treatable by hormone therapy. Most women dislike bumps and indentations on their bodies and prefer to be as smooth as they possibly can. Additionally, because hope springs eternal, much has been written about cellulite and its causes and many treatments ― either by topical or oral administration - have been promoted.

With respect to active compositions for treating human skin in general and for fighting cellulites in particular, reference is made to international patent application **WO 00/037039 A1** (Pentapharm) disclosing a topical composition comprising: (a) conjugated linoleic acid, and/or derivatives thereof comprising conjugated linoleic acid moieties, in which at least 1 % by weight of the conjugated linoleic acid and/or moieties is present as the trans 10, cis 12 isomer, and (b) a dermatologically acceptable carrier.

In addition, international patent applications **WO 00/003039 A1 and WO 00/0037040 A1** (Unilever) are directed to a topical composition comprising: (a) conjugated linoleic acid, and/or derivatives thereof comprising conjugated linoleic acid moieties, in which (a1) at least 1 % by weight of the conjugated linoleic acid and/or moieties is present as the t10,c12-isomer, or (a2) at least 50% by weight of the conjugated linoleic acid and/or moieties, is present as the c9,t11-isomer; , and (b) a dermatologically acceptable carrier.

Finally, international patent application **WO 01/017498 A1** (Ghisalberti) claims the use of conjugated linoleic acid (CLA) for the topical treatment of fatty deposits and cellulite and to new topical compositions and to cosmetic and dermatological topical compositions for the treatment of fatty deposits and cellulite comprising CLA as well as kits comprising CLA for said treatment.

As a matter of fact, none of the products which can be find in the market so far have shown a really satisfying result. Therefore, the underlying problem of the present invention has been to provide new cosmetic or pharmaceutical compositions for improved treatment of human skin in general and more particular for fighting cellulites. Such compositions should allow to chose between topical and oral administration.

### Description of the invention

The present invention claims cosmetic and/or pharmaceutical compositions comprising
(a) physiologically active fatty acids, their salts and their esters, and
(b) extracts of *Castanea sativa* or its active principles.

Surprisingly it has been found that mixtures of physiologically active fatty acids, mainly conjugated linoleic acid or its derivatives, and extracts of horse chestnuts, mainly those extracts which are rich in β-escin, show synergistic properties in strengthening the female connective tissue and fighting the causes for cellulites.

### Physiologically active fatty acids, their salts and their esters

A common criteria for fatty acids with physiological activity, which represent component (b), is a fat chain having a sufficient number of carbon atoms providing a lipophilic behaviour that allows the molecule pass through the gastrointestinal tract of the body and a sufficient number of double bonds. Therefore, said fatty acids usually comprise 18 to 26 carbon atoms and 2 to 6 double bonds.

In a first embodiment of the present invention conjugated linoleic acid (CLA) or its alkaline or alkaline earth salts and esters, preferably esters with lower aliphatic alcohols having 1 to 4 carbon atoms - or their glycerides, specially their triglycerides come into account. Conjugated linoleic acid (CLA) represents a commercially available product which usually is obtained by base-catalysed isomerisation of sunflower oil or their respective alkyl esters and subsequent isomerisation in the presence of enzymes. CLA is an acronym used for positional and geometric isomers deriving from the essential fatty acid linoleic acid (LA, cis-9,cis-12-octadecadienoic acid, 18:2n-6). From a physiological point of view the use of the *cis*-9,*trans-*11 isomer according to the present invention is of special importance having at least 30, preferably at least 50 and most preferably at least 80 % b.w. of said *cis*-9,*trans*-11 isomer - calculated on the total CLA content of the crude mixture. In addition, it has been found advantageous if the content of the *trans*-10,*cis*-12 isomer is at most 45, preferably at most 10 % b.w. and most preferably is less than 1 % b.w., and the sum of 8,10-, 11,13- and *trans,trans-*isomers in total is less than 1 % b.w. ― again calculated on the total CLA content. Such products can be found in the market for example under the trademark Tonalin® CLA-80 (Cognis).

In a second embodiment also so-called omega-3 fatty acids can come into account, which typically comprise 18 to 26, preferably 20 to 22 carbon atoms and at least 4 and up to 6 double bonds. Also these molecules are very well known from the art and can be obtained by standard methods of organic chemistry, for example via transesterification of fish oils, followed by urea precipitation of the alkyl esters thus obtained and a final extraction using non-polar solvents as described in the German patent DE 3926658 C2 (Norsk Hydro). Fatty acids thus obtained are rich in omega-3 (all-Z)-5,8,11,14,17-eicosapentanoic acid (EPA) C 20 : 5 and (all-Z)-4,7,10,13,16,19-docosahexanoic acid (DHA) C 22 : 6. Such products can be found in the market under the trademark Omacor® (Pronova).

In a third embodiment also linoleic acid, vaccinic acid (trans 11-octadecenoic acid) or cis-hexadecenoic acid (obtained for example from the plant *Thunbergia alata)* can be used.

In addition said physiologically active fatty acid esters can not only be used in form of their lower alkyl esters or glycerides, an additional well preferred embodiment of the present invention relates to compositions comprising esters of said fatty acids with sterols. Like glycerides sterol esters are easily resorbed and splitted by the human body, however, a significant advantage comes from the fact that the cleavage of the ester bond releases a second molecule with health promoting properties. To avoid unclearities, the phrases "sterol","stanol" and "sterin" shall be used as synonyms defining steroids showing a single hydroxyl group linked to the C-3. In addition sterols, which consist of 27 to 30 carbon atoms, may show a double bond, preferably in 5/6 position. According to the present invention esters of CLA or omega-3 fatty acids with β-sitosterol or its hydrogenation product β-sitostanol are preferred.

### Extracts of Castanea sativa and its active principles

Main ingredients of extracts horse chestnuts (*Castanea sativa*), which represent coponent (b), are saponins and escin, which is a mixture of two glycosides, whose aglycons are derived from proteoescigenin, while the sugars represent either glucoronic acid of two molecules D-glucose. Said glycosides differ in the acyl groups in the C22-position.

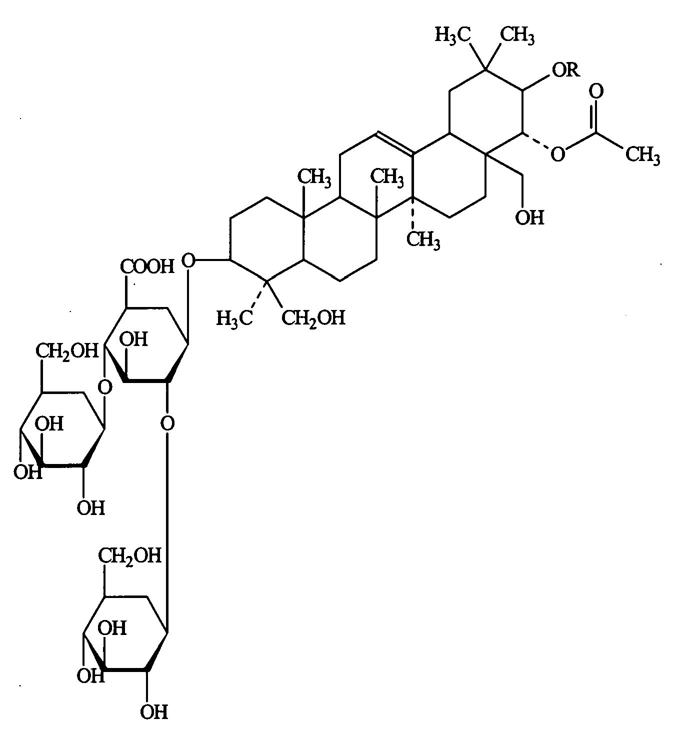

### R = tiglic acid or angelic acid

While α-escin represents an amorphous powder, which melts between 225 and 227 °C and is easily soluble in water, β-escin (which is also called flogencyl) forms flakes, which are practically water-insoluble, but can be dissolved in alcohol.

### Cosmetic and/or pharmaceutical compositions

The compositions according to the present invention may be administered either topical or oral and can comprise component (a) and component (b) in a weight ratio of 99 to 1 to 50 : 50 and more particularly 95 : 10 to 75 : 25. The highest synergistic effects, however, are observed at ratios of 92 : 8 to 80 : 20. In general, the compositions can be used in a concentration of up to about 10, particularly 0.5 to 8 and more particularly 1 to 2 % b.w. - calculated the final composition. One percent, however, has been found to be particularly suitable.

In order to support the anti-cellulite and skin lightening effect the compositions may comprise further plant extracts or their active principles, for example chosen from the plants selected from the group consisting of *Ginkgo biloba, Oleacea europensis, Glyzyrrhiza glabra, Vaccin-ium myrtillus, Trifolium pratense, Litchi sinensis, Vitis, vinifera, Brassica oleracea, Punica granatum, Petroselinium crispum, Centella asiatica, Passiflora incarnata, Medicago sativa, Valeriana officinalis, Salix alba* and *Harpagophytum procumbens.*

### Macro- or micro-encapsulation

In a special embodiment of the present invention said compositions are macro- or micro-encapsulated. "Microcapsules" are understood to be spherical aggregates with a diameter of about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("micro-sponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semisynthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolysates, sucrose and waxes. Semisynthetic membrane materials are inter alia chemically modified celluloses, more particularly cellulose esters and ethers, for example cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone. Examples of known microcapsules are the following commercial products (the membrane material is shown in brackets) *Hallcrest Microcapsules* (gelatin, gum arabic), *Coletica Thalaspheres* (maritime collagen), *Lipotec Millicapseln* (alginic acid, agar agar), *Induchem Unispheres* (lactose, microcrystalline cellulose, hydroxypropylmethyl cellulose), *Unicerin C30* (lactose, microcrystalline cellulose, hydroxypropylmethyl cellulose), *Kobo Gly-cospheres* (modified starch, fatty acid esters, phospholipids), *Softspheres* (modified agar agar), *Kuhs Probiol Nanospheres* (phospholipids) and *Primaspheres* or *Primasponges* (chitosan, anionic polymers).

### Industrial application

A further embodiment of the present invention relates to the use of the mixtures for making cosmetic and/or pharmaceutical compositions, in which they may be present in amounts of from 0.1 to 30, preferably 1 to 25 and more preferably 2 to 5 % b.w. - calculated on the final composition. Finally, also claimed is the use of the mixtures for making a medicament for fighting cellulites.

### Cosmetic and/or pharmaceutical preparations

The preparations according to the invention may contain surfactants, emulsifiers, superfatting agents, pearlizing waxes, consistency factors, polymers, silicone compounds, waxes, stabilizers, primary and secondary sun protection agents, biogenic agents, film formers, swelling agents, hydrotropes, preservatives, solubilizers, complexing agents, perfume oils, dyes and the like as additional auxiliaries and additives.

### Surfactants

Other preferred auxiliaries and additives are anionic and/or amphoteric or zwitterionic surfactants. Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulfates, protein fatty acid condensates (particularly wheat-based vegetable products) and alkyl (ether) phosphates. If the anionic surfactants contain polyglycol ether chains, they may have a conventional homolog distribution although they preferably have a narrow-range homolog distribution. Typical examples of amphoteric or zwitterionic surfactants are alkylbetaines, alkylamidobetaines, aminopropionates, aminoglycinates, imidazolinium betaines and sulfobetaines. The surfactants mentioned are all known compounds. Information on their structure and production can be found in relevant synoptic works, cf. for example **J. Falbe (ed.), "Surfactants in Consumer Products", Springer Verlag, Berlin, 1987, pages 54 to 124** or **J. Falbe (ed.), "Katalysatoren, Tenside und Mineralöladditive (Catalysts, Surfactants and** Mineral Oil Additives)", Thieme Verlag, Stuttgart, 1978, pages 123-217. The percentage content of surfactants in the preparations may be from 0.1 to 10% by weight and is preferably from 0.5 to 5% by weight, based on the preparation.

### Emulsifiers

Other surfactants may also be added to the preparations as emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

### • Partial glycerides

Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

### • Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

### • Polyglycerol esters

Typical examples of suitable polyglycerol esters are Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) and Polyglyceryl Polyricinoleate (Admul® WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Typical anionic emulsifiers are aliphatic C₁₂₋₂₂ fatty acids, such as palmitic acid, stearic acid or behenic acid for example, and C₁₂₋₂₂ dicarboxylic acids, such as azelaic acid or sebacic acid for example.

Other suitable emulsifiers are zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkyl-aminopropionic acids and alkylaminoacetic acids containing around 8 to 18 carbon atoms in the alkyl group. Particularly preferred ampholytic surfactants are N-cocoalkylaminopro-pionate, cocoacylaminoethyl aminopropionate and C_{12/18} acyl sarcosine.

### Superfatting agents

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

### Consistency factors

The consistency factors mainly used are fatty alcohols or hydroxy fatty alcohols containing 12 to 22 and preferably 16 to 18 carbon atoms and also partial glycerides, fatty acids or hydroxy fatty acids. A combination of these substances with alkyl oligoglucosides and/or fatty acid N-methyl glucamides of the same chain length and/or polyglycerol poly-12-hydroxystearates is preferably used.

### Thickening agents

Suitable thickeners are polymeric thickeners, such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grünau), quaternized wheat polypeptides, polyethyleneimine, cationic silicone polymers such as, for example, amodimethicone, copolymers of adipic acid and dimethylaminohydroxypropyl diethylenetriamine (Cartaretine®, Sandoz), copolymers of acrylic acid with dimethyl diallyl ammonium chloride (Merquat® 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar®CBS, Jaguar®C-17, Jaguar®C-16 of Celanese, quaternized ammonium salt polymers such as, for example, Mirapol® A-15, Mirapol® AD-1, Mirapol® AZ-1 of Miranol.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlizing waxes

Suitable pearlizing waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy- substituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et **al. in Cosm. Toil. 91, 27 (1976).**

### Waxes

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

### Stabilizers

Metal salts of fatty acids such as, for example, magnesium, aluminium and/or zinc stearate or ricinoleate may be used as stabilizers.

### Primary sun protection factors

Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances:
- 3-benzylidene camphor or 3-benzylidene norcamphor and derivatives thereof, for example 3-(4-methylbenzylidene)-camphor;
- 4-aminobenzoic acid derivatives, preferably 4-(dimethylamino)-benzoic acid-2-ethylhexyl ester, 4-(dimethylamino)-benzoic acid-2-octyl ester and 4-(dimethylamino)-benzoic acid amyl ester;
- esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
- esters of salicylic acid, preferably salicylic acid-2-ethylhexyl ester, salicylic acid-4-isopropylbenzyl ester, salicylic acid homomenthyl ester;
- derivatives of benzophenone, preferably 2-hydroxy-4-methoxybenzophenone, 2-hydroxy-4-methoxy-4'-methylbenzophenone, 2,2'-dihydroxy-4-methoxybenzophenone;
- esters of benzalmalonic acid, preferably 4-methoxybenzalmalonic acid di-2-ethylhexyl ester;
- triazine derivatives such as, for example, 2,4,6-trianilino-(p-carbo-2'-ethyl-1'-hexyloxy)-1,3,5-triazine and Octyl Triazone or Dioctyl Butamido Triazone (Uvasorb® HEB);
- propane-1,3-diones such as, for example, 1-(4-tert.butylphenyl)-3-(4'-methoxyphenyl)-propane-1,3-dione;
- ketotricyclo(5.2.1.0)decane derivatives.

Suitable water-soluble substances are
- 2-phenylbenzimidazole-5-sulfonic acid and alkali metal, alkaline earth metal, ammonium, alkylammonium, alkanolammonium and glucammonium salts thereof;
- sulfonic acid derivatives of benzophenones, preferably 2-hydroxy-4-methoxybenzophenone-5-sulfonic acid and salts thereof;
- sulfonic acid derivatives of 3-benzylidene camphor such as, for example, 4-(2-oxo-3-bornylidenemethyl)-benzene sulfonic acid and 2-methyl-5-(2-oxo-3-bornylidene)-sulfonic acid and salts thereof.

Typical UV-A filters are, in particular, derivatives of benzoyl methane such as, for example, 1-(4'-tert.butylphenyl)-3-(4'-methoxyphenyl)-propane-1,3-dione, 4-tert.butyl-4'-methoxydibenzoyl methane (Parsol® 1789) or 1-phenyl-3-(4'-isopropylphenyl)-propane-1,3-dione and the enamine compounds (BASF). The UV-A and UV-B filters may of course also be used in the form of mixtures. Particularly favourable combinations consist of the derivatives of benzoyl methane, for example 4-tert.butyl-4'-methoxydibenzoyl methane (Parsol® 1789) and 2-cyano-3,3-phenylcinnamic acid-2-ethylhexyl ester (Octocrylene®), in combination with esters of cinnamic acid, preferably 4-methoxycinnamic acid-2-ethylhexyl ester and/or 4-methoxycinnamic acid propyl ester and/or 4-methoxycinnamic acid isoamyl ester. Combinations such as these are advantageously combined with water-soluble filters such as, for example, 2-phenylbenzimidazole-5-sulfonic acid and alkali metal, alkaline earth metal, ammonium, alkylammonium, alkanolammonium and glucammonium salts thereof.

### Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example □-carotene, □-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, □-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homo- cysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

### Biogenic agents

In the context of the invention, biogenic agents are, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, for example prune extract, bambara nut extract, and vitamin complexes.

### Film formers

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

### Hydrotropes

In addition, hydrotropes, for example ethanol, isopropyl alcohol or polyols, may be used to improve flow behavior. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 dalton; '
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane; trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Complexing agents

The complexing agents used may be selected from EDTA, NTA, phosphonic acids, Triton B, turpinal and phenacetin. In addition, reducing agents such as, for example, ascorbic acid, sodium sulfate, sodium thiosulfate and the like may be present. Suitable alkalizing agents are ammonia, monoethanolamines, (L) arginine, AMP, etc.

### Perfume oils

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### Dyes

Suitable dyes are any of the substances suitable and approved for cosmetic purposes as listed, for example, in the publication **"Kosmetische Färbemittel"** of the Farbstoffkommission der Deutschen Forschungsgemeinschaft, Verlag Chemie, Weinheim, 1984, pages 81 to 106. Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. These dyes are normally used in concentrations of 0.001 to 0.1% by weight, based on the mixture as a whole.

The total percentage content of auxiliaries and additives may be from 1 to 50% by weight and is preferably from 5 to 40% by weight, based on the particular composition. The compositions may be produced by standard hot or cold processes.

### Examples

### Preparation examples

Moisturising creams according to the composition of table 1 were prepared using the PIT technology. Creams 1 and 2 are according to the invention, creams C1 and C2 were used for comparison.

**Table 1 Moisturising creams**

| **Composition** | **Cream C1** | **Cream C2** | **Cream 1** | **Cream 2** |
|---|---|---|---|---|
| **Emulgade® SE** Glyceryl Sterate (and) Ceteareth 12/20 (and) Cetearyl Alcohol (and) Cetyl Palmitate | 5,0 | 5,0 | 5,0 | 5,0 |
| **Cetiol® SN** Cetearyl Isononanoate | 3,0 | 3,0 | 3,0 | 3,0 |
| **Cetiol® V** Decyl Oleate | 3,0 | 3,0 | 3,0 | 3,0 |
| **Nutrilan® Elastin E20** Hydrolyzed Elastin | 2,0 | 2,0 | 2,0 | 2,0 |
| **Tonalin® CLA** Conjugated Linoleic acid | - | 1,0 | 0,9 | 0,8 |
| **Herbalia® Chestnut** Horse chestnuts extract | - | - | 0,1 | 0,2 |
| **Hydagen® CMF** Chitosan | 1,0 | 1,0 | 1,0 | 1,0 |
| **Glycerin** (86 Gew.-%ig) | 3,0 | 3,0 | 3,0 | 3,0 |
| **Water, Preservatives** | ad to 100 | | | |

### Application examples

### Topical application of CLA/horse chestnuts extract mixture

9 female subjects were selected based on their cellulite intensity in the thigh area having a bilateral symmetry. Subjects with grades 1 and 2 cellulite were chosen, as a 5-point grading scale was used to rate the cellulite severity of each subject. The scale ranged from 0 to 4 (0 = no cellulite; 1 = small bumps or depressions; 2 = striations and bumps; 3 = pronounced lumpiness of the skin and striations; 4 = all of the above plus hard sub-surface nodules). The subjects were divided in 3 groups of 3 individuals each, and instructed to apply in the right thigh the cream of Comparative Examples C1 and C2 and Inventive Examples 1 and 2 respectively. The subject were taught to carried out the application two times a day, at morning and at night, during 2 months. Afterwards the cellulite condition were evaluated according Smith WP **(Cosmetics & Toiletries, June 1995, p61-70),** by comparison of the right thigh versus left thigh. The results are summarised in Table 2.

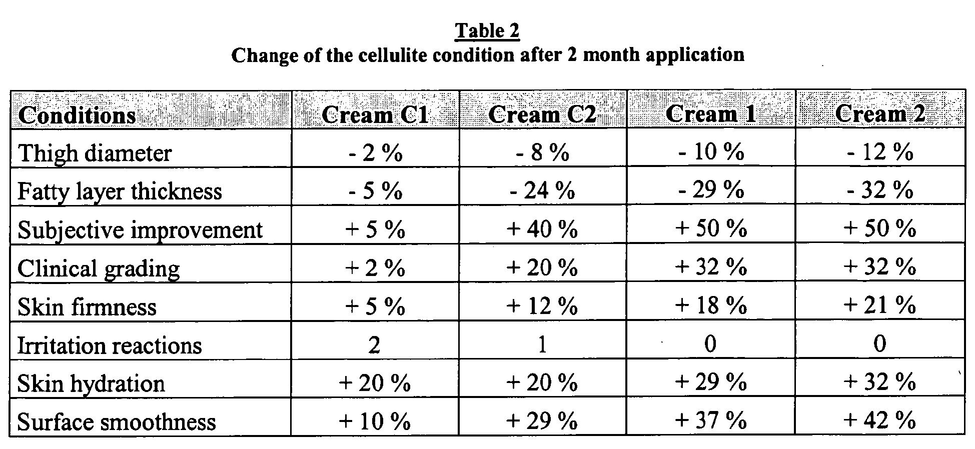

The results indicate that the creams comprising CLA in combination with horse chestnuts extracts result in a superior skin status compared with creams comprising CLA alone.

## Claims

**1.** Cosmetic and/or pharmaceutical compositions comprising
(a) physiologically active fatty acids, their salts and their esters, and
(b) extracts of *Castanea sativa* or its active principles.

**2.** Compositions according to claim 1, **characterised in that** said physiologically active fatty acids (component a) comprise 18 to 26 carbon atoms and 2 to 6 double bonds.

**4.** Compositions according to any of the claims 1 to 3, **characterised in that** component (a) represents esters of said fatty acids with glycerol or sterol.

**5.** Compositions according to any of the claims 1 to 4, **characterised in that** component (a) represents conjugated linoleic acid or omega-3 fatty acids.

**6.** Compositions according to any of the claims 1 to 5, **characterised in that** component (b) represents β-escin or an extract of *Castanea sativa* rich in β-escin.

**7.** Compositions according to any of the claims 1 to 6, **characterised in that** they comprise component (a) and (b) in weight ratios of from 99 : 1 to 50 : 50.

**8.** Compositions according to any of the claims 1 to 7, **characterised in that** said mixtures are macro- or micro-encapsulated.

**9.** Use of mixtures according to claim 1 for making cosmetic and/or pharmaceutical compositions.

**10.** Use of mixtures according to claim 1 for making a medicament for fighting cellulites.
